# NEUE EUROPÄISCHE PATENTSCHRIFT

(11) **EP 1 485 094 B2**
(45) Veröffentlichungstag und Bekanntmachung des Hinweises auf die Entscheidung über den Einspruch: **25.03.2020**
(45) Hinweis auf die Patenterteilung: 04.07.2012
(21) Anmeldenummer: 03743368.7
(22) Anmeldetag: 03.03.2003
(51) Int. Cl.: A61K 9/16, A61K 9/50, A61K 31/4439, A61P 7/02

(54) **ORAL ZU APPLIZIERENDE DARREICHUNGSFORM FÜR 3- [(2-{[4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO] -PROPIONSÄURE-ETHYLESTER ODER DESSEN SALZE**
DOSAGE FORM FOR ORAL ADMINISTRATION OF 3-[(2-{[4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO] PROPIONIC ACID ETHYL ESTER OR ITS SALTS
FORME POSOLOGIQUE POUR ADMINISTRATION PAR VOIE ORALE DE L'ETHYLESTER D'ACIDE 3-[(2-{[4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO] PROPIONIQUE OU SES SELS

(30) Priorität: 07.03.2002 DE 10209985; 30.09.2002 DE 10245624
(43) Veröffentlichungstag der Anmeldung: 15.12.2004
(60) Teilanmeldung: 07115663.2
(73) Patentinhaber: Boehringer Ingelheim International GmbH, 55216 Ingelheim am Rhein (DE)
(72) Erfinder: BRAUNS, Ulrich, 88400 Biberach (DE); HAUEL, Norbert, 88433 Schemmerhofen (DE)
(74) Vertreter: Hoffmann Eitle
(86) Internationale Anmeldenummer: PCT/EP2003/002141
(87) Internationale Veröffentlichungsnummer: WO 2003/074056

(56) Entgegenhaltungen:
- WO-A-98/37075
- WO-A-03/007984
- US-A- 4 438 091
- BERKOWITZ SCOTT D: "Antithrombotic therapy after prosthetic cardiac valve implantation: Potential novel antithrombotic therapies." AMERICAN HEART JOURNAL, Bd. 142, Nr. 1, Juli 2001 (2001-07), Seiten 7-13, XP001146897 ISSN: 0002-8703
- MUNGALL DENNIS: "BIBR-1048 Boehringer Ingelheim." CURRENT OPINION IN INVESTIGATIONAL DRUGS (LONDON, ENGLAND: 2000) ENGLAND JUN 2002, Bd. 3, Nr. 6, Juni 2002 (2002-06), Seiten 905-907, XP001147306 ISSN: 1472-4472
- DATABASE WPI 15 Mai 2007 Derwent Publications Ltd., London, GB; AN 1983-767185 'Acid-added cinnarizine compsns. - for use as cerebral vasodilator by oral admin.' & JP 58 134033 A (FUJISAWA PHARM CO LTD) 10 August 1983

## Beschreibung

Die Erfindung betrifft eine oral zu applizierende Darreichungsform für den Wirkstoff 3-[(2-{[4-(Hexyloxycarbonylamino-imino-methyl)-phenylamino]-methyl}-methyl-1*H* benzimidazol-5-carbonyl)-pyridin-2-yl-amino]-propionsäure-ethylester und dessen pharmakologisch verträglichen Salze. Dieser Wirkstoff mit der chemischen Formel 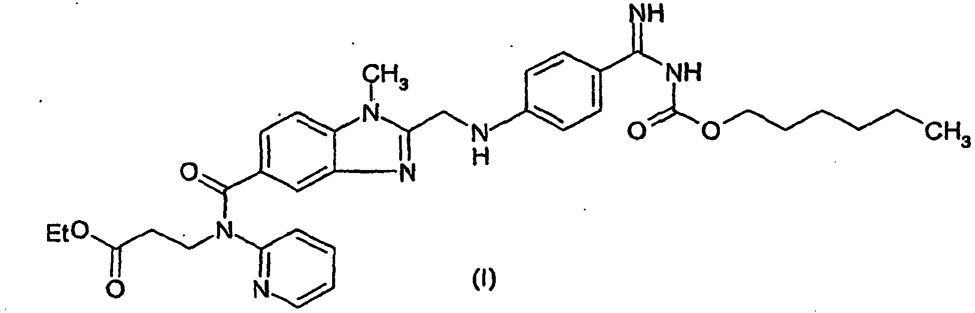 ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxycarbönylamidino)phenyl]-amino-methyl]-benzimidazol-5-yl-carbonsäure-*N*-(2-pyridyl)-*N*-(2-ethoxycarbonylethyl)-amid bekannt Bei der Verbindung der Formel I handelt es sich um ein Doppel-Prodrug der Verbindung 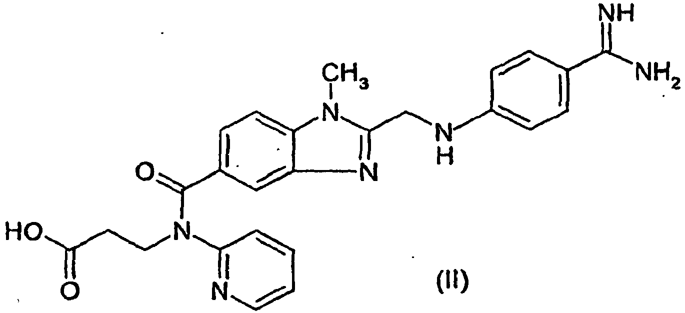 d.h. die Verbindung der Formel I wird erst im Körper in die eigentlich wirksame Verbindung, nämlich die Verbindung der Formel II, umgewandelt. Hauptindikationsgebiet der Verbindung der chemischen Formel I ist die postoperative Prophylaxe von tiefen Venenthrombosen.

Die US 4,438,091 beschreibt eine pharmazeutische Zusammensetzung für Bromhexin mit verzögerter Wirkstofffreisetzung, welche beispielsweise aus Starterkemen aus Weinsäure besteht, die mit einer pulvrigen Bromhexin-Mischung besprüht und anschließend mit einer Schicht versehen werden, die die Freisetzung in Magen und Darm steuert.

In der JP 58 134033 werden diverse orale Darreichungsformen (Granulat, Kapseln, Tablette, Pulver, Dragees) für Cinnarizin beschrieben, in welchen die stabile Absorption von Cinnarizin unabhängig von Änderungen des Magen-pH durch den Zusatz von sauren Substanzen wie beispielsweise Weinsäure oder Zitronensäure erreicht wird.

Aufgabe der Erfindung ist es, eine verbesserte Formulierung zur oralen Anwendung für die Verbindung der Formel I (die im folgenden auch als "Wirkstoff" bezeichnet wird) bereitzustellen. Zur Lösung dieser Aufgabe wird erfindungsgemäß eine pharmazeutische Zusammensetzung zur oralen Applikation des Wirkstoffs 3-[(2-{[4-(Hexyloxycarbonylamino-imino-methyl)-phenylamino]-methyl}-1-methyl-1*H*-benzimidazol-5-carbonyl)-pyridin-2-yl-aminoJ-propionsäure-ethylester oder eines seiner pharmazeutisch akzeptablen Salze vorgeschlagen, die die folgenden Bestandteile umfasst: ein annähernd sphärisches Kernmaterial, das aus der pharmazeutisch akzeptablen organischen Säure mit einer Wasserlöslichkeit von > 1 g / 250 ml bei 20°C ausgewählt aus einer Gruppe bestehend aus Weinsäure, Fumarsäure, Bernsteinsäure, Zitronensäure, Äpfelsäure, Glutaminsäure und Asparaginsäure oder eines von deren Hydraten oder sauren Salzen besteht oder diese enthält, eine binde- und gegebenenfalls trennmittelhaltige Wirkstoffschicht, die das Kernmaterial umschließt, wobei das Kernmaterial und die Wirkstoffschicht durch eine Isolierschicht bestehend aus einem wasserlöslichen Polymer voneinander getrennt sind. Von den vorstehend genannten pharmazeutisch akzeptablen organischen Säuren sind im Sinne dieser Erfindung besonders geeignet Weinsäure, Fumarsäure, Bernsteinsäure und Zitronensäure.

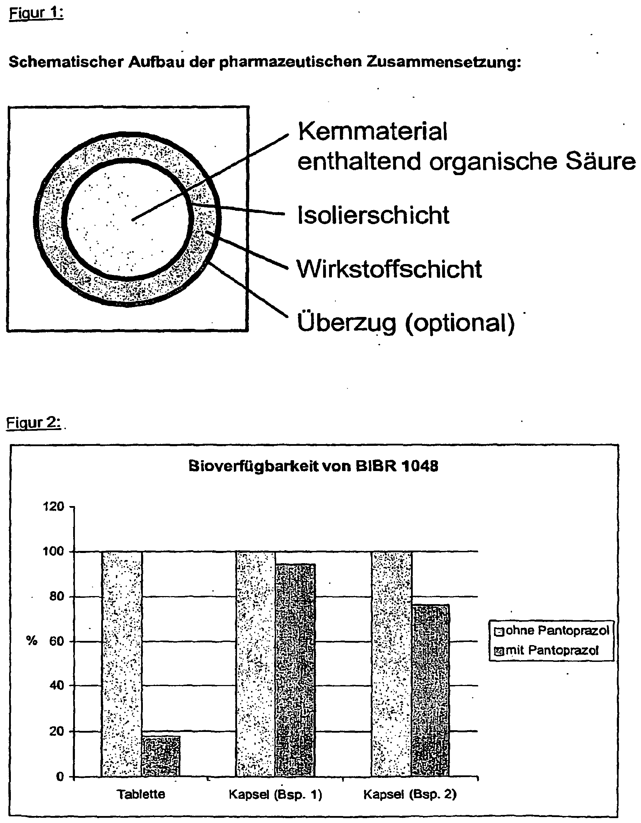

Eine bevorzugte Ausführungsform der Erfindung ist eine multipartikuläre Darreichungsform, in der die einzelnen Partikel wie in Figur 1 aufgebaut sind. Figur 1 stellt den schematischen Aufbau der pharmazeutischen Zusammensetzung anhand eines Schnitts durch ein für die Herstellung der erfindungsgemäßen pharmazeutischen Zusammensetzung geeignetes Pellet dar. Der annähend kugelförmige/ sphärische Kernbereich dieses Pellets enthält/besteht aus der pharmazeutisch akzeptablen organischen Säure. Dann folgt eine Schicht, die den Säure-Kem von der wirkstoffhaltigen Schicht trennt, die sog. Isolierschicht. Die Isolierschicht wiederum ist von der ebenfalls kugelschafenförmigen Wirkstoffschicht umgeben, die ihrerseits von einem Überzug umschlossen sein kann, der die Abriebfestigkeit und die Lagerstabilität der Pellets erhöht.

Ein Vorteil der so aufgebauten Darreichungsform ist die räumliche Trennung von organischer Säure und Wirkstoff durch die Isolierschicht. Ein weiterer Vorteil des oben beschriebenen Aufbaus der Pellets ist die Tatsache, dass die organische Säure erst nach Applikation der Darreichungsform in Lösung geht und dann ein saures Mikroklima erzeugt, in dem sich der Wirkstoff auflösen kann.

Als Kemmaterial wird eine pharmazeutisch akzeptable organische Säuren mit einer Wasserlöslichkeit von > 1 g / 250 ml bei 20° C ausgewählt aus der Gruppe bestehend aus Weinsäure, Fumarsäure, Bernsteinsäure, Zitronensäure, Äpfelsäure, Glutaminsäure und Asparaginsäure einschließlich deren Hydraten und sauren Salzen, verwendet, der gegebenenfalls ein geringer Anteil von 1 bis 10 Gew.-%, bevorzugt 3 bis 6 Gew.-% eines geeignete Bindemittels zugesetzt wird. Die Verwendung eines Bindemittels ist z.B. dann erforderlich, wenn die Säurestarter in einem Kessel-aufbau-verfahren hergestellt werden. Bei Verwendung eines Extrusions- oder Sphäronisierungs-Verfahrens benötigt man an Stelle von Bindemitteln andere technologische Hilfsstoffe, beispielsweise mikrokristalline Cellulose. Es ist auch denkbar, reine (100 %-ige) Säure als Startmaterial zu verwenden, wenn man diese in hinreichend enger Korngrößenverteilung beschaffen kann. Als pharmazeutisch akzeptable organische Säure werden bevorzugt Weinsäure, Fumarsäure, Bernsteinsäure oder Zitronensäure eingesetzt; besonders bevorzugt ist Weinsäure. Als Bindemittel können Gummi arabicum oder ein partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere verwendet werden; bevorzugt ist Gummi arabicum. Das sphärische Kemmaterial hat vorzugsweise einen mittleren Durchmesser von 0,4-1,5 mm. Der Gehalt der pharmazeutisch akzeptablen organischen Säure liegt üblicherweise zwischen 30 und 100 % im Kernmaterial, was einem Anteil von zwischen 20 und 90 %, vorzugsweise von zwischen 20 und 80 % im fertigen Pellet (d.h. in der pharmazeutischen Zusammensetzung) entspricht. Zur Erhöhung der Lagerstabilität des Fertigproduktes ist es vorteilhaft, das Kernmaterial vor dem Auftrag des Wirkstoffes mit einer Isolierschicht basierend auf einem wasserlöslichen, pharmazeutisch akzeptablen Polymer zu beschichten. Als solches wasserlösliches Polymer kommen beispielsweise Gummi arabicum oder ein partial-oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere in Frage. Bevorzugt wird Gummi arabicum oder eine Hydroxypropylmethylcellulose verwendet. Gegebenenfalls kann die Beschichtung mit dem wasserlöslichen, pharmazeutisch akzeptablen Polymer unter Zusatz geeigneter Weichmacher, Trennmittel und Pigmente erfolgen, wie beispielsweise Triethylcitrat, Tributylcitrat, Triacetin, Polyethylenglykole (Weichmacher), Talkum, Kieselsäure (Trennmittel), Titandioxid oder Eisen-oxidpigmente (Pigmente).

Die Wirkstoffschicht enthält den Wirkstoff 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester (BIBR 1048) oder eines seiner pharmazeutisch akzeptablen Salze sowie Binde- und gegebenenfalls Trennmittel. Ein bevorzugtes Salz des Wirkstoffs ist das Mesylat (Methansulfonat) der Verbindung der Formel I. Als Bindemittel können beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus *N*-Vinylpyrrolidon und Vinylacetat oder Kombinationen dieser Polymere verwendet werden. Vorzugsweise wird Hydroxypropylcellulose oder Copolymerisate aus *N*-Vinylpyrrolidon und Vinylacetat eingesetzt. Der Zusatz von Trennmitteln wie z.B. Talkum oder Kieselsäure dient dazu, ein Zusammenwachsen der Partikel während des Prozesses zu verhindern. Der Wirkstoffgehalt beträgt 5 bis 60 %, vorzugsweise 10 bis 50 % der pharmazeutischen Zusammensetzung.

Die optionale äußerste Schicht, die zur Verminderung eines erhöhten Abriebs bei der Abfüllung in Kapseln und/oder zur Erhöhung der Lagerstabilität dient, besteht aus pharmazeutisch üblichen Filmbildnern, Weichmachern und gegebenenfalls Pigmenten. Als Filmbildner können beispielsweise Hydroxypropylcellulose, Hydroxypropylmethylcellulose, Methylcellulose, Polymerisate und Copolymerisate der Acryl- und Methacrylsäure und deren Estern, oder Kombinationen dieser Polymere verwendet werden. Als Weichmacher kommen unter anderem Triethylcitrat, Tributylcitrat, Triacetin oder Polyethylenglykole in Frage. Als Pigmente können z.B. Titandioxid oder Eisenoxidpigmente eingesetzt werden. Bevorzugt besteht der äußere Überzug aus Hydroxypropylmethylcellulose und / oder Methylcellulose, gegebenenfalls unter Zusatz von Polyethylenglykolen als Weichmacher.

Die Pellets können nach dem im folgenden beschriebenen Verfahren hergestellt werden:
Das säurehaltige Kemmaterial besteht entweder aus Kristallen der jeweils zur Verwendung kommenden organischen Säure oder vorteilhafter aus annähernd sphärischen Partikeln in der gewünschten Größe mit einem hohen Gehalt an organischer Säure, die mittels Verfahren hergestellt werden können, die in der pharmazeutischen Technologie bekannt und etabliert sind. In Frage kommen insbesondere der Aufbau des Kernmaterials in Kesselverfahren, auf Pelletiertellem, oder mittels Extrusion / Sphäronisierung. Anschließend kann das so erhaltenen Kemmaterial durch Sieben in Fraktionen mit dem gewünschten Durchmesser geteilt werden. Geeignetes Kernmaterial hat einen mittleren Durchmesser von 0,4 bis 1,5 mm, bevorzugt von 0,6 bis 0,8 mm.

Auf dieses säurehaltige Kernmaterial wird zunächst die Isolierschicht aufgetragen. Dies kann durch übliche Verfahren, z.B. durch Auftragen einer wäßrigen Dispersion des wasserlöslichen, pharmazeutisch akzeptablen Polymers gegebenenfalls unter Zusatz von Weichmachern, Trennmitteln und / oder Pigmenten in der Wirbelschicht, in Dragierkesseln oder in üblichen Filmcoatinganlagen, geschehen. Falls erforderlich, kann anschließend erneut gesiebt werden.

Daran anschließend wird der Wirkstoff aus einer bindemittel- und ggf. trennmittelhaltigen Dispersion aufgetragen. Das flüchtige Dispersionsmittel wird während des Prozesses und/oder daran anschließend durch Trocknen entfernt. Als Bindemittel in der Dispersion kann beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus N-Vinylpyrrolidon und Vinylacetat oder Kombinationen dieser Polymere verwendet werden. Vorzugsweise wird Hydroxypropylcellulose oder Copolymerisate aus N-Vinylpyrrolidon und Vinylacetat eingesetzt. Als Trennmittel eignen sich z.B. Talkum oder Kieselsäure; vorzugsweise wird Talkum verwendet. Als Dispersionsmittel kommen beispielsweise Ethanol, 2-Propanol, Aceton oder Mischungen dieser Lösungsmittel miteinander oder mit Wasser, bevorzugt 2-Propanol in Frage. Der Wirkstoffauftrag auf das Kernmaterial kann mittels in der pharmazeutischen Technologie bekannter und etablierter Verfahren z.B. in Dragierkesseln, konventionellen Filmcoatinganlagen oder in der Wirbelschicht erfolgen. Anschließend kann erneut ein Siebprozess durchgeführt werden.

Zur Verminderung eines erhöhten Abriebs bei der Abfüllung in Kapseln oder zur Erhöhung der Lagerstabilität kann das System abschließend noch mit einer Schicht aus pharmazeutisch üblichen Filmbildnern, Weichmachern und ggf. Pigmenten überzogen werden. Dies kann mit Hilfe von üblichen Verfahren erfolgen, wie sie bereits bei der Beschreibung des Aufbringens der Isolierschicht erwähnt wurden.

Bei Verwendung von Kemmaterial mit einem mittleren Durchmesser von 0,4 -1,5 mm werden durch das oben beschriebene Verfahren wirkstoffhaltige Pellets erhalten, die anschließend beispielsweise in Hartkapseln abgefüllt werden können. Dazu wird eine der Dosierung entsprechende Vielzahl dieser Einheiten auf Standard-Kapselfüllmaschinen in Hartkapseln gefüllt. Als geeignete Hartkapseln kommen beispielsweise Hartgelatinekapseln oder Hartkapseln aus Hydroxypropylmethylcellulose (HPMC) in Frage; bevorzugt sind HPMC-Kapseln. Der Wirkstoffgehalt der pharmazeutischen Zusammensetzung beträgt 5 bis 60 %, vorzugsweise 10 bis 50 %; der Gehalt der pharmazeutisch akzeptablen organischen Säure liegt üblicherweise zwischen 20 und 90 %, vorzugsweise zwischen 20 und 80 %.

Soweit nicht anders vermerkt, handelt es sich bei den Prozentangaben stets um Gewichtsprozente. Sämtliche Angaben über den Wirkstoffgehalt beziehen sich, sofern nicht anders angegeben, auf die Wirkstoffbase der Formel I (nicht auf ein bestimmtes Salz).

### Klinische Prüfungen

Bei den ersten Probanden-Versuchen mit konventionellen Tabletten enthaltend die Verbindung der Formel I war festgestellt worden, daß hochvariable Plasmaspiegel bis hin zu vereinzelten Malabsorptionen auftraten. Die Variabilität der Plasmaspiegelverläufe ist nach Applikation der Verbindung der Formel I als oral applizierte Lösung deutlich niedriger; eine Malabsorption wurde hierbei nicht beobachtet. Untersuchungen haben gezeigt, dass die Verbindung der Formel I in Wasser bei niedrigen pH-Werten verhältnismäßig gut löslich ist, während sie bei pH-Werten über 5 gemäß der Definition des europäischen Arzneibuches praktisch unlöslich ist. Daher wurde den Probanden in einem Behandlungsast der klinischen Prüfungen Pantoprazol verabreicht, welches dazu dient, einen erhöhten Magen-pH hervorzurufen.

Beispielsweise wurden die pharmazeutischen Zusammensetzungen gemäß der Beispiele 1 und 2 auf ihre Bioverfügbarkeit im Vergleich zu einer konventionellen Tablette hin getestet.

Dazu wurde die gemäß Beispiel 1 hergestellte Formulierung mit einem Gehalt an Wirkstoffbase von 50 mg pro Kapsel an insgesamt 15 Probanden im Hinblick auf ihre Bioverfügbarkeit klinisch geprüft. In einem Behandlungsast erhielten die Probanden die Zusammensetzung nüchtern per os (= orale Gabe) ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. (= zwei mal täglich) per os vorbehandelt; die Behandlung mit Pantoprazol wurde während der Verabreichung der erfindungsgemäßen Formulierung fortgeführt.

Das Ausmaß der Absorption wurde über die quantitative Bestimmung der Urinausscheidung des wirksamen Metaboliten der Formel II ermittelt

Die relative Bioverfügbarkeit nach Vorbehandlung mit Pantoprazol lag im Vergleich zur Applikation ohne vorhergehende Vorbehandlung im Mittel bei 94 %.

Unter vergleichbaren Applikationsbedingungen liegt die relative Bioverfügbarkeit (basierend auf der Fläche unter der Plasmakonzentrations-Zeit Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

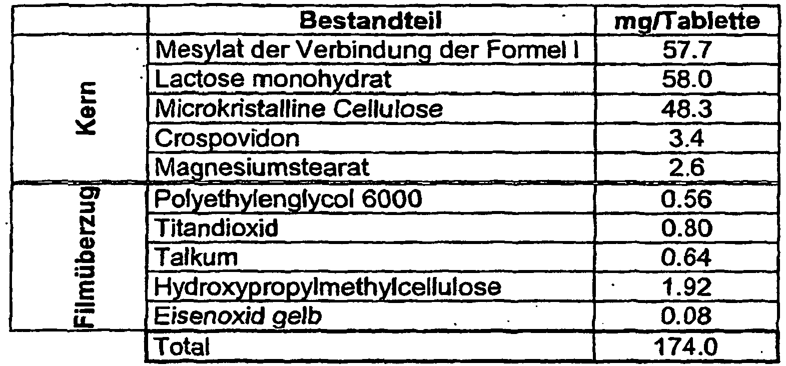

Die relative Bioverfügbarkeit wurde durch Verwendung der erfindungsgemäßen Formulierung also ca. um den Faktor 5 verbessert.

Die gemäß Beispiel 2 hergestellte Formulierung mit einem Gehalt an Wirkstoffbase von 50 mg pro Kapsel wurde ebenfalls an insgesamt 15 Probanden im Hinblick auf ihre Bioverfügbarkeit klinisch geprüft. In einem Behandlungsast erhielten die Probanden die Zusammensetzung nüchtern per os ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. per os vorbehandelt; die Behandlung mit Pantoprazol wurde während der Verabreichung der erfindungsgemäßen Formulierung fortgeführt.

Das Ausmaß der Absorption wurde über die quantitative Bestimmung der Urinausscheidung des wirksamen Metaboliten der Formel II ermittelt.

Die relative Bioverfügbarkeit nach Vorbehandlung mit Pantoprazol lag im Vergleich zur Applikation ohne vorhergehende Vorbehandlung im Mittel bei 76 %.

Unter vergleichbaren Applikationsbedingungen liegt die relative Bioverfügbarkeit (basierend auf der Fläche unter der Plasmakonzentrations-Zeit Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

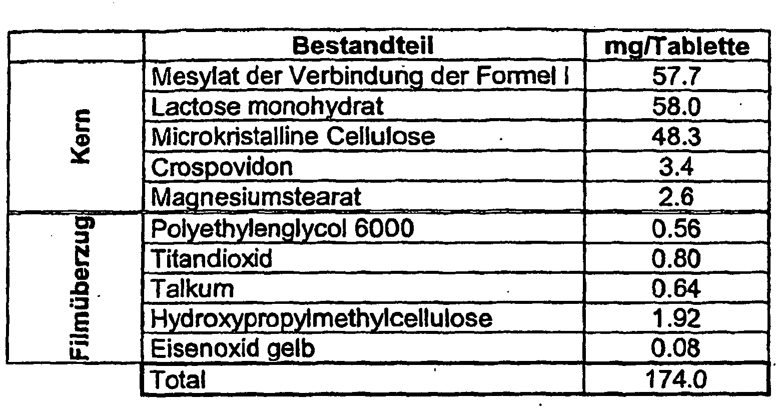

Durch die Verwendung der erfindungsgemäßen Formulierung wurde die relative Bioverfügbarkeit des Wirkstoffs gegenüber herkömmlicher Formulierungen also ca. um den Faktor 4 verbessert. Die Bioverfügbarkeit der beiden erfindungsgemäßen Formulierungen im Vergleich zur oben beschriebenen Tablette mit bzw. ohne gleichzeitige Gabe von Pantoproazol ist in Figur 2 graphisch dargestellt.

Die klinischen Prüfung zeigen einen weiteren Vorteil des erfindungsgemäßen Darreichungsform enthaltend die Verbindung der Formel I, der darin besteht, eine ausreichende, gegenüber einer konventionellen pharmazeutischen Zubereitung verbesserte sowie vom Magen-pH weitgehend unabhängige Bioverfügbarkeit des Wirkstoffs zu gewährleisten, Schwankungen der Bioverfügbarkeit des Wirkstoffs zu vermindern und Malabsorptionen zu verhindern. Eine weitere vorteilhafte Eigenschaft der erfindungsgemäßen pharmazeutischen Zusammensetzung ist ihre Eignung für alle Patienten, also auch für solche Personen, bei denen der Magen-pH durch normale physiologische Variabilität, durch eine Krankheit oder durch eine Komedikation mit Arzneimitteln, die den Magen-pH erhöhen, erhöht ist.

Die Dosierung beträgt bei oraler Gabe zweckmäßigerweise 25 bis 300 mg der Wirkstoffbase (pro Kapsel), vorzugsweise 50 bis 200 mg, besonders bevorzugt 75 bis 150 mg der Wirkstoffbase, jeweils 1 bis 2 mal täglich.

Das bevorzugte Säure-Wrkstoffverhältnis beträgt ca. 0,9 : 1 bis ca. 4 : 1, besonders bevorzugt ist ein Verhältnis von ca. 1:1 und von ca. 3:1. Bevorzugt wird mindestens 1 Equivalent Säure pro Mol der Verbindung der Formel I verwendet. Die Obergrenze von ca. 4:1 (Säure zu Wirkstoff), wird in der Regel durch die maximal akzeptable Größe der Darreichungsform bei den gewünschten Dosierungen bestimmt (Menge der Pellets pro Kapsel).

Die nachfolgenden Beispiele sollen die Erfindung näher erläutern:

### Beispiel 1

| | Prozentuale Zusammensetzung | | | | pro Kapsel [mg] | pro Kapsel [mg] |
|---|---|---|---|---|---|---|
| | Kernmaterial | Isolierschicht | Wirkstoff schicht | Gesamt | | |
| Weinsäure | 61,3 | - | - | 61,3 | 176,7 | 353,4 |
| Gummi arabicum | 3,1 | 2,8 | | 5,9 | 17,0 | 34,0 |
| Talkum | - | 5,6 | 3,2 | 8,8 | 25,4 | 50,7 |
| Hydroxypropylcellulose | - | - | 4,0 | 4,0 | 11,5 | 23,1 |
| Wirkstoff (Mesylat der Verbindung der Formel I) | - | - | 20,0 | 20,0 | 57,7* | 115,3** |
| Summe | | | | 100,0 | 288,3 | 576,5 |
| *) entspricht 50 mg der Verbindung der Formel 1 (Wirkstoffbase) | | | | | | |
| **) entspricht 100 mg der Verbindung der Formel 1 (Wirkstoffbase) | | | | | | |

### a) Aufbau von weinsäurehaltigem Kernmaterial

### Zusammensetzung:

| | |
|---|---|
| Gummi arabicum | 1 Gewichtsteil |
| Weinsäure | 20 Gewichtsteile |

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekemmaterial wird anschließend im rotierenden Kessel bei einer Zulufttemperatur von 60° - 80° C nachgetrocknet.

Das Kemmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

### b) Isolierung des weinsäurehaltigen Kernmaterials

### Zusammensetzung:

| | |
|---|---|
| Weinsäurehaltiges Kemmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

### c) Aufbau der Wirkstoffschicht

### Zusammensetzung:

| | |
|---|---|
| Isoliertes weinsäurehaltiges Kernmaterial | 91 Gewichtsteile |
| Hydroxypropylcellulose | 5 Gewichtsteile |
| Talkum | 4 Gewichtsteile |
| Wirkstoff (Mesylat von BIBR 1048) | 25 Gewichtsteile |

In 168 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kemmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

### d) Abfüllung in Kapseln

Eine jewels 50 bzw. 100 mg Wirkstoffbase enthaltende Menge an wirkstoffhaltigen Pellets wird mittels einer Kapselfüllmaschine in Hartgelatinekapseln oder HPMC-Kapseln der Größe 1 bzw. der Größe 0 elongated abgefüllt.

### Beispiel 2

| | Prozentuale Zusammensetzung | | | | pro Kapsel [mg] | pro Kapsel [mg] |
|---|---|---|---|---|---|---|
| | Kernmaterial | Isolierschicht | Wirkstoff schicht | Gesamt | | |
| Weinsäure | 38,5 | - | - | 38,5 | 55,5 | 166,5 |
| Gummi arabicum | 1,9 | 1,7 | | 3,6 | 5,2 | 15,6 |
| Talkum | - | 3,5 | 6,4 | 9,9 | 14,3 | 42,8 |
| Hydroxypropylcellulose | - | - | 8,0 | 8,0 | 11,5 | 34,6 |
| Wirkstoff (Mesylat der Verbindung der Formel I) | - | - | 40,0 | 40,0 | 57,7* | 173,0** |
| Summe | | | | 100,0 | 144,2 | 432,5 |
| *) entspricht 50 mg der Verbindung der Formel 1 (Wirkstoffbase) | | | | | | |
| **) entspricht 150 mg der Verbindung der Formel 1 (Wirkstoffbase) | | | | | | |

### a) Aufbau von weinsäurehaltigem Kernmaterial

### Zusammensetzung:

| | |
|---|---|
| Gummi arabicum | 1 Gewichtsteil |
| Weinsäure | 20 Gewichtsteile |

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekemmaterial wird anschließend im rotierenden Kessel bei einer Zulufttemperatur von 60° - 80° C nachgetrocknet.

Das Kemmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

### b) Isolierung des weinsäurehaltigen Kernmaterials

### Zusammensetzung:

| | |
|---|---|
| Weinsäurehaltiges Kemmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kemmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

### c) Aufbau der Wirkstoffschicht

### Zusammensetzung:

| | |
|---|---|
| Isoliertes weinsäurehaltiges Kernmaterial | 57 Gewichtsteile |
| Hydroxypropylcellulose | 10 Gewichtsteile |
| Talkum | 8 Gewichtsteile |
| Wirkstoff (Mesylat von BIBR 1048) | 50 Gewichtsteile |

In 335 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kemmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

### d) Abfüllung in Kapseln

Eine jeweils 50 bzw. 150 mg Wirkstoffbase enthaltende Menge an wirkstoffhaltigen Pellets wird mittels einer Kapselfüllmaschine in Hartgelatinekapseln oder HPMC-Kapseln der Größe 2 bzw. der Größe 0 abgefüllt.

### Beispiel 3

### Herstellung von 3-[(2-{[4-(Hexyloxycarbonylamino-imino-methyl)-phenylaminol-methyl}-1-methyl-1H-benzimidazol-5-carbonyl)-pyridin-2-yl-amino]-propionsäure-ethylester-Methansulfonat

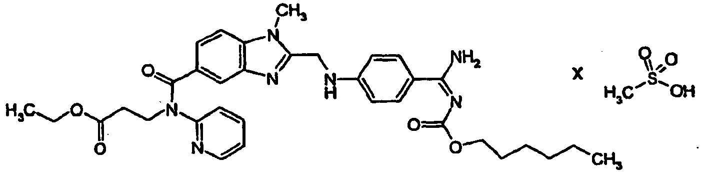

Zu einer Lösung von 3139 mg (5.0 mMol) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base (wie in der WO 98/37075 beschrieben hergestellt), in 250 ml Essigsäureethylester wurde eine Lösung von 5.0 mmol Methansulfonsäure in 25 ml Essigsäureethylester unter Rühren bei Raumtemperatur tropfenweise zugegeben. Nach wenigen Minuten begann das Produkt auszukristallisieren. Es wurde noch eine Stunde lang bei Raumtemperatur und eine weitere unter Eiskühlung gerührt, dann der Niederschlag abgesaugt, mit ca. 50 ml Essigester und 50 ml Diethylether gewaschen und bei 50°C im Umluft-Trockenschrank getrocknet.
Ausbeute: 94% der Theorie
Schmelzpunkt: 178 - 179°C
C₃₄H₄₁N₇O₅ x CH₄SO₃ (723.86)

| | | | | |
|---|---|---|---|---|
| Elementar-Analyse:ber.: | C 58.07% | H 6.27% | N 13.55% | S 4.43% |
| gef:: | 58.11% | 6.30% | 13.50% | 4.48% |

## Patentansprüche

1. Pharmazeutische Zusammensetzung zur oralen Applikation des Wirkstoffs 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester oder eines seiner pharmazeutisch akzeptablen Salze, umfassend
ein annähernd sphärisches Kernmaterial, das aus der pharmazeutisch akzeptablen organischen Säure mit einer Wasserlöslichkeit von > 1 g / 250 ml bei 20 °C ausgewählt aus einer Gruppe bestehend aus Weinsäure, Fumarsäure, Bemsteinsäure, Zitronensäure, Äpfelsäure, Glutaminsäure und Asparaginsäure oder eines von deren Hydraten oder sauren Salzen besteht oder diese enthält,
eine binde- und gegebenenfalls trennmittelhaltige Wirkstoffschicht, die das Kemmaterial umschließt,
wobei das Kemmaterial und die Wirkstoffschicht durch eine Isolierschicht bestehend aus einem wasserlöslichen Polymer voneinander getrennt sind.

2. Pharmazeutische Zusammensetzung nach Anspruch 1, **dadurch gekennzeichnet, dass** die pharmazeutisch akzeptable organische Säure Weinsäure, Fumarsäure, Zitronensäure oder Bernsteinsäure ist.

3. Pharmazeutische Zusammensetzung nach Anspruch 2, **dadurch gekennzeichnet, dass** die pharmazeutisch akzeptable organische Säure Weinsäure ist.

4. Pharmazeutische Zusammensetzung nach einem der Ansprüche 1 bis 3, bei der der Gehalt an 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester oder dessen Salzen in der pharmazeutischen Zusammensetzung 5 bis 60 % beträgt.

5. Pharmazeutische Zusammensetzung nach den Ansprüchen 1 bis 4, bei der der Gehalt an pharmazeutisch akzeptabler organischer Säure 20 bis 90 % beträgt.

6. Pharmazeutische Zusammensetzung nach einem der Ansprüche 1 bis 5, bei der das Bindemittel aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat oder aus Kombinationen dieser Polymere besteht.

7. Pharmazeutische Zusammensetzung nach einem der Ansprüche 1 bis 6, bei der das Kemmaterial eine mittlere Partikelgröße von 0,4 bis 1,5 mm aufweist.

8. Pharmazeutische Zusammensetzung nach einem der Ansprüche 1 bis 7, bei der das wasserlösliche Polymer aus Gummi arabicum oder einem partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder aus Kombinationen dieser Polymere besteht.

9. Pharmazeutische Zusammensetzung nach einem der Ansprüche 1 bis 8, bei der das wirkstoffhaltige Produkt in Hartkapseln abgefüllt wird.

10. Pharmazeutische Zusammensetzung nach Anspruch 9, bei der das wirkstoffhaltige Produkt in Hydroxypropylmethylcellulose-Kapseln abgefüllt wird.

11. Pharmazeutische Zusammensetzung nach einem der Ansprüche 1 bis 10, bei der 3-[(2-{[4-(Hexyloxycarbonylaminoimino-methyl)-phenylamino]-methyl}-1-methyl-1H-benzimidazol-5-carbonyl)-pyridin-2-yl-amino]propionsäure-ethylester Mesylat als Wirkstoff verwendet wird.

12. Verfahren zur Herstellung einer pharmazeutischen Zusammensetzung gemäß den Ansprüchen 1 bis 11, umfassend die Schritte:
a) Aufbau des Kemmaterials aus einer oder mehreren pharmazeutisch akzeptablen organischen Säure(n) mit einer Wasserlöslichkeit von > 1 g / 250 ml bei 20 °C ausgewählt aus einer Gruppe bestehend aus Weinsäure, Fumarsäure, Bernsteinsäure, Zitronensäure, Äpfelsäure, Glutaminsäure und Asparaginsäure oder eines von deren Hydraten oder sauren Salzen gegebenenfalls unter Zusatz von Bindemitteln oder anderer technologischer Hilfsstoffe, in Kesselverfahren, auf Pelletiertellem oder mittels Extrusion / Sphäronisierung,
b) Auftragen einer Isolierschicht bestehend aus einem oder mehreren wasserlöslichen, pharmazeutisch akzeptablen Polymeren gegebenenfalls unter Zusatz von Weichmachern, Trennmitteln und/oder Pigmenten auf das Kenmaterial,
c) Auftragen des Wirkstoffs aus einer bindemittel- und gegebenenfalls trennmittelhaltigen Dispersion und gleichzeitiges und/oder anschließendes Trocknen zur Entfernung des Dispersionsmittels,
d) gegebenenfalls Aufbringen eines Überzugs aus Filmbildnem, Weichmachern und gegebenenfalls Pigmenten und
e) Abfüllung der so erhaltenen wirkstoffhaltigen Pellets in Hartkapseln.

## Claims

1. Pharmaceutical composition for oral administration of the active substance ethyl 3-[(2-{[4-(hexyloxycarbonylamino-imino-methyl)-phenylamino]-methyl}-1-methyl-1*H-*benzimidazole-5-carbonyl)-pyridin-2-yl-amino]-propionate or one of the pharmaceutically acceptable salts thereof, comprising
a substantially spherical core material which consists of or contains the pharmaceutically acceptable organic acid having a water solubility of > 1 g / 250 ml at 20°C selected from among tartaric acid, fumaric acid, succinic acid, citric acid, malic acid, glutamic acid or aspartic acid or one of the hydrates or acid salts thereof,
an active substance layer containing binder and optionally separating agent, which encloses the core material,
the core material and the active substance layer being separated from one another by an insulating layer consisting of a water-soluble polymer.

2. Pharmaceutical composition according to claim 1, **characterised in that** the pharmaceutically acceptable organic acid is tartaric acid, fumaric acid, citric acid or succinic acid.

3. Pharmaceutical composition according to claim 2, **characterised in that** the pharmaceutically acceptable organic acid is tartaric acid.

4. Pharmaceutical composition according to one of claims 1 to 3, wherein the content of ethyl 3-[(2-{[4-(hexyloxycarbonylamino-imino-methyl)-phenylamino]-methyl}-1-methyl-1*H-*benzimidazole-5-carbonyl)-pyridin-2-yl-amino]-propionate or the salts thereof in the pharmaceutical composition is 5 to 60 %.

5. Pharmaceutical composition according to claims 1 to 4, wherein the content of pharmaceutically acceptable organic acid is 20 to 90 %.

6. Pharmaceutical composition according to one of claims 1 to 5, wherein the binder consists of the group of hydroxypropylcelluloses, hydroxypropylmethylcelluloses, methylcelluloses, hydroxyethylcelluloses, carboxymethylcelluloses, polyvinylpyrrolidones, copolymers of N-vinylpyrrolidone and vinyl acetate or of combinations of these polymers.

7. Pharmaceutical composition according to one of claims 1 to 6, wherein the core material has an average particle size of 0.4 to 1.5 mm.

8. Pharmaceutical composition according to one of claims 1 to 7, wherein the water-soluble polymer consists of gum arabic or a partially or totally synthetic polymer from the group of hydroxypropylcelluloses, hydroxypropylmethylcelluloses, methylcelluloses, hydroxyethylcelluloses, carboxymethylcelluloses, polyvinylpyrrolidones, copolymers of N-vinylpyrrolidone and vinyl acetate, or of combinations of these polymers.

9. Pharmaceutical composition according to one of claims 1 to 8, wherein the product containing the active substance is packed into hard capsules.

10. Pharmaceutical composition according to claim 9, wherein the product containing the active substance is packed into hydroxypropylmethylcellulose capsules.

11. Pharmaceutical composition according to one of claims 1 to 10, wherein ethyl 3-[(2-{[4-(hexyloxycarbonylamino-imino-methyl)-phenylamino]-methyl}-1-methyl-1*H-*benzimidazole-5-carbonyl)-pyridin-2-yl-amino]-propionate mesylate is used as the active substance.

12. Process for preparing a pharmaceutical composition according to claims 1 to 11, comprising the steps of:
a) forming the core material from one or more pharmaceutically acceptable organic acid(s) having a water solubility of > 1 g / 250 ml at 20°C selected from among tartaric acid, fumaric acid, succinic acid, citric acid, malic acid, glutamic acid or aspartic acid or one of the hydrates or acid salts thereof, optionally with the addition of binders or other technological adjuvants, by pan methods, on pelleting plates or by extrusion / spheronisation,
b) applying to the core material an insulating layer consisting of one or more water-soluble, pharmaceutically acceptable polymers optionally with the addition of plasticisers, separating agents and/or pigments,
c) applying the active substance from a dispersion which contains a binder and optionally separating agents and simultaneously and/or subsequently drying to eliminate the dispersant,
d) optionally applying a coating of film-forming agents, plasticisers and optionally pigments and
e) packing the active substance-containing pellets thus obtained into hard capsules.

## Revendications

1. Composition pharmaceutique pour l'administration orale de la substance active éthylester d'acide 3-[(2-{[4-(hexyloxycarbonylamino-imino-méthyl)-phénylamino]-méthyl}-1-méthyl-1H-benzimidazol-5-carbonyl)-pyridin-2-yl-amino]-propionique ou de l'un de ses sels pharmaceutiquement acceptables, comprenant
un matériau de noyau pratiquement sphérique qui est constitué d'un acide organique pharmaceutiquement acceptable avec une solubilité aqueuse > 1 g/250 ml à 20 °C choisi dans le groupe comprenant l'acide tartrique, l'acide fumarique, l'acide succinique, l'acide citrique, l'acide malique, l'acide glutamique et l'acide aspartique ou l'un de leurs hydrates ou sels acides ou contient ceux-ci,
une couche de substance active comprenant un liant et éventuellement un agent anti-agglomérant qui entoure le matériau de noyau,
dans laquelle le matériau de noyau et la couche de substance active sont séparés par une couche isolante constituée d'un polymère hydrosoluble.

2. Composition pharmaceutique selon la revendication 1, **caractérisée en ce que** l'acide organique pharmaceutiquement acceptable est l'acide tartrique, l'acide fumarique, l'acide citrique ou l'acide succinique.

3. Composition pharmaceutique selon la revendication 2, **caractérisée en ce que** l'acide organique pharmaceutiquement acceptable est l'acide tartrique.

4. Composition pharmaceutique selon l'une des revendications 1 à 3, dans laquelle la teneur en éthylester d'acide 3-[(2-{[4-(hexyloxy-carbonylamino-imino-méthyl)-phénylamino]-méthyl}-1-méthyl-1H-benzimidazol-5-carbonyl)-pyridin-2-yl-amino]-propionique ou ses sels dans la composition pharmaceutique est de 5 à 60 %.

5. Composition pharmaceutique selon les revendications 1 à 4, dans laquelle la teneur en acide organique pharmaceutiquement acceptable est de 20 à 90 %.

6. Composition pharmaceutique selon l'une des revendications 1 à 5, dans laquelle le liant est constitué du groupe des hydroxypropylcelluloses, des hydroxypropylméthylcelluloses, des méthylcelluloses, des hydroxyéthylcelluloses, des carboxyméthylcelluloses, des polyvinylpyrrolidones, des copolymères de N-vinylpyrrolidone et d'acétate de vinyle ou de combinaisons de ces polymères.

7. Composition pharmaceutique selon l'une des revendications 1 à 6, dans laquelle le matériau de noyau présente une taille de particule moyenne de 0,4 à 1,5 mm.

8. Composition pharmaceutique selon l'une des revendications 1 à 7, dans laquelle le polymère hydrosoluble comprend la gomme arabique ou un polymère partiellement ou complètement synthétique du groupe des hydroxypropylcelluloses, des hydroxypropylméthylcelluloses, des méthylcelluloses, des hydroxyéthylcelluloses, des carboxyméthylcelluloses, des polyvinylpyrrolidones, des copolymères de N-vinylpyrrolidone et d'acétate de vinyle ou une combinaison de ces polymères.

9. Composition pharmaceutique selon l'une des revendications 1 à 8, dans laquelle le produit contenant la substance active est conditionné dans des capsules dures.

10. Composition pharmaceutique selon la revendication 9, dans laquelle le produit contenant la substance active est conditionné dans des capsules d'hydroxypropylméthylcellulose.

11. Composition pharmaceutique selon l'une des revendications 1 à 10, dans laquelle le mésylate d'éthylester d'acide 3-[(2-{[4-(hexyloxy-carbonylamino-imino-méthyl)-phénylamino]-méthyl}-1-méthyl-1H-benzimidazol-5-carbonyl)-pyridin-2-yl-amino]-propionique est utilisé comme substance active.

12. Procédé de production d'une composition pharmaceutique selon les revendications 1 à 11, comprenant les étapes suivantes :
a) la formation du matériau de noyau à partir d'un ou plusieurs acide(s) organiques pharmaceutiquement acceptable(s) avec une solubilité aqueuse > 1 g/250 ml à 20 °C choisi(s) dans le groupe comprenant l'acide tartrique, l'acide fumarique, l'acide succinique, l'acide citrique, l'acide malique, l'acide glutamique et l'acide aspartique ou l'un de leurs hydrates ou sels acides, éventuellement en ajoutant des liants ou d'autres adjuvants technologiques, dans un procédé en cuve, sur des particules agglomérées ou par extrusion/sphéronisation,
b) l'application d'une couche isolante constituée d'un ou plusieurs polymère(s) hydrosoluble(s) pharmaceutiquement acceptable(s) éventuellement en ajoutant des plastifiants, des anti-agglomérants et/ou des pigments sur le matériau de noyau,
c) l'application de la substance active à partir d'une dispersion contenant l'agent liant et éventuellement un anti-agglomérant et séchage simultané et/ou ultérieur pour éliminer l'agent de dispersion,
d) éventuellement, l'application d'un enrobage constitué d'agents filmogènes, de plastifiants et éventuellement de pigments et
e) le conditionnement des granulés contenant la substance active ainsi obtenue dans des capsules dures.
